# EUROPEAN PATENT APPLICATION

(11) **EP 4 467 192 A1**
(43) Date of publication of application: **27.11.2024**
(21) Application number: 24177966.9
(22) Date of filing: 24.05.2024
(51) Int. Cl.: A61N 5/10, G21K 1/093, G21K 5/04, H05H 7/04, H05H 13/10

(54) **ELECTRON BEAM TREATMENT DEVICE, METHOD FOR ELECTRON BEAM FLASH THERAPY, AND SYSTEM FOR FLASH THERAPY**

(30) Priority: 24.05.2023 CN 202310595658; 30.01.2024 WO PCT/CN2024/074785
(71) Applicant: Shanghai United Imaging Healthcare Co., Ltd., Shanghai 201807 (CN)
(72) Inventor: ZHOU, Liuyuan, Shanghai, 201807 (CN); HE, Shoubo, Shanghai, 201807 (CN); WANG, Peng, Shanghai, 201807 (CN); PAN, Gang, Shanghai, 201807 (CN); NI, Cheng, Shanghai, 201807 (CN)
(74) Representative: Viering, Jentschura & Partner mbB Patent- und Rechtsanwälte

(57) **Abstract**

An electron beam treatment device (900), a method for electron beam flash therapy, and a system for flash therapy are provided. The electron beam treatment device (900) includes an electron beam emitter (9), a first deflection member (30), and an annular deflection mechanism (40). The electron beam emitter (9) is configured to configured to emit a high-energy electron beam. The first deflection member (30) can perform first deflection processing on the high-energy electron beam passing through the first deflection member (30). The annular deflection mechanism (40) is configured to receive the high-energy electron beam deflected by the first deflection member (30), and perform second deflection processing on the high-energy electron beam, so that the high-energy electron beam is emitted towards a target region.

## Description

### CROSS-REFERENCE TO RELATED APPLICATIONS

This application claims priority to Chinese patent application No. 202310595658.0, filed on May 24, 2023, titled "ELECTRON BEAM FLASH THERAPY DEVICE, ELECTRON BEAM FLASH THERAPY METHOD, AND COMPUTER READABLE STORAGE MEDIUM", and international patent application No. PCT/CN 2024/074785, filed on January 30, 2024, titled "RADIATION TREATMENT SYSTEMS AND METHODS".

### TECHNICAL FIELD

The present invention generally relates to the field of medical devices, and in particular, to an electron beam treatment device, a method for electron beam flash therapy, and a system for flash therapy.

### BACKGROUND

Flash therapy is a therapeutic solution of radiation therapy for a tumor. A dosage rate of flash therapy is many times higher than that of current radiation therapy, and accurate dosage can be provided.

At present, in a conventional flash therapy solution that uses a high-energy electron beam, a high-gradient linear accelerator is generally used to generate a high-energy electron beam required for the flash therapy, and then multi-angle exposure to the tumor is implemented by using a rotatable rack. Although the foregoing solution can generate the high-energy electron beam applicable to the flash therapy, the foregoing solution is limited to a structural characteristic of the high-gradient linear accelerator, so that a device to which the solution is applied is not only large in size, but also high in construction costs and subsequent maintenance costs. In addition, in the related art, a mechanical manner of rotating a gantry is used to adjust a position of an isocenter at which the high-energy electron beam is finally converged, so as to adapt to different tumor positions of different patients. However, the gantry is large, so operation is inconvenient and adjustment efficiency is low.

### SUMMARY

According to various embodiments of the present invention, an electron beam treatment device, a method for electron beam flash therapy, and a system for flash therapy are provided.

In a first aspect, an electron beam treatment device is provided, configured to generate and deliver a high energy electron beam that is applicable to flash therapy. The electron beam treatment device includes an electron beam emitter, a first deflection member, and an annular deflection mechanism. The electron beam emitter is configured to emit a high-energy electron beam. The first deflection member is configured to be capable of performing first deflection processing on the high-energy electron beam. The annular deflection mechanism is configured to receive the high-energy electron beam deflected by the first deflection member, and perform second deflection processing on the high-energy electron beam, so that the high-energy electron beam is emitted towards a target region.

In an embodiment, energy of the high-energy electron beam is in a range of 100MeV to 200MeV.

The electron beam emitter includes an electron beam generator and an electron cyclotron, the electron beam generator is configured to generate an electron beam, and the electron cyclotron is configured to receive the electron beam from the electron beam generator, and perform cyclotron acceleration processing on the electron beam, so that the electron beam is formed into the high-energy electron beam.

In an embodiment, the electron cyclotron includes an acceleration cavity and two groups of vector magnets, the two groups of vector magnets are symmetrically disposed at both sides of the acceleration cavity and are configured to guide the electron beam emitted from the acceleration cavity to re-enter the acceleration cavity, and the acceleration cavity is capable of performing acceleration processing on the electron beam in the acceleration cavity.

In an embodiment, the electron cyclotron includes a lead-out magnet, the lead-out magnet is disposed on a cyclic path of the electron beam in the electron cyclotron, the lead-out magnet is configured to lead out the high-energy electron beam formed after acceleration, and a position of the lead-out magnet is capable of being adjusted relative to the electron cyclotron.

In an embodiment, the electron cyclotron includes at least one of a race-track microtron, a betatron, or a petal-shaped accelerator.

In an embodiment, a generated magnetic field strength of the first deflection member is capable of being adjusted when the first deflection member is powered on, so that the high-energy electron beam passing through the first deflection member is capable of entering the annular deflection mechanism at different angles.

In an embodiment, the electron beam treatment device further includes a second deflection member, the second deflection member is configured to receive the high-energy electron beam emitted from the annular deflection mechanism and perform third deflection processing on the high-energy electron beam, and a generated magnetic field strength of the second deflection member is capable of being adjusted when the second deflection member is powered on.

In an embodiment, the first deflection member includes either or both of a vector magnet and a deflection resonant cavity.

In an embodiment, the electron beam treatment device further includes a movable supporting device configured for supporting a target subject, and the movable supporting device is configured to adjust a position of the target region relative to the annular deflection mechanism during an electron beam flash therapy, so that the target region is irradiated by the high-energy electron beam from different directions.

In an embodiment, the movable supporting device is rotatable so that the target region is able to be irradiated by the high-energy electron beam from any angle.

In an embodiment, a position of the target region relative to the electron beam treatment device is capable of being changed during a treatment.

In an embodiment, the movable supporting device is rotatable to change an orientation of the target region.

In an embodiment, an irradiation direction of the electron beam towards the target region is substantially fixed.

In an embodiment, an irradiation direction of the electron beam towards the target region is changeable.

In an embodiment, the electron beam treatment device further includes a beam regulator configured to split the electron beam into a plurality of sub-beams transmitting along different directions, the electron beam treatment device includes a plurality of groups of the first deflection member and the annular deflection mechanism, which are configured to adjust the sub-beams, respectively, to generate a plurality of high-energy electron beams, and the plurality of high-energy electron beams are capable of irradiating the target region from different directions.

In an embodiment, the beam regulator further includes a beam splitting element and a plurality of constraint elements. The beam splitting element is configured to split the electron beam into the plurality of sub-beams. The plurality of constraint elements are configured to constrain transmission directions of the plurality of sub-beams, so that each of the plurality of sub-beams enters into one of the plurality of groups of the first deflection member and the annular deflection mechanism.

In an embodiment, the electron beam treatment device further includes a converter and a collimator, the converter is configured to convert the electron beam into a third radiation beam, and the collimator is configured to adjust the third radiation beam to align to the target region.

In a second aspect, a method for electron beam flash therapy is provided. The method for electron beam flash therapy includes: emitting a high-energy electron beam by an electron beam emitter, directing the high-energy electron beam to a first deflection member, performing first deflection processing on the high-energy electron beam by the first deflection member, directing the high-energy electron beam processed by the first deflection processing to an annular deflection mechanism, and performing second deflection processing on the high-energy electron beam by the annular deflection mechanism, so that the high-energy electron beam is emitted towards a target region.

In a third aspect, a system for flash therapy is provided. The system for flash therapy includes an electron beam treatment device configured to generate and deliver a high-energy electron beam that is applicable to flash therapy. The electron beam treatment device includes an electron beam emitter, a first deflection member, and an annular deflection mechanism. The electron beam emitter is configured to emit a high-energy electron beam. The first deflection member is capable of performing first deflection processing on the high-energy electron beam. The annular deflection mechanism is configured to receive the high-energy electron beam deflected by the first deflection member, and perform second deflection processing on the high-energy electron beam, so that the high-energy electron beam is emitted towards a target region.

In an embodiment, the system further includes a second radiation treatment device configured to emit a second radiation beam towards the target region for radiation treatment, and the high-energy electron beam and the second radiation beam are configured for irradiating the target region from different directions.

In an embodiment, energy levels of the high-energy electron beam and the second radiation beam are determined based on a position of the target region relative to the electron beam treatment device and the second radiation treatment device.

In a fourth aspect, a computer readable storage medium is provided. An instruction is stored on the computer readable storage medium, and the instruction implements the above method for electron beam flash therapy when executed by a processor.

Details of one or more embodiments of the present invention are set forth in the following accompanying drawings and description. Other features, objectives, and advantages of the present invention become obvious with reference to the specification, the accompanying drawings, and the claims.

### BRIEF DESCRIPTION OF THE DRAWINGS

In order to more clearly illustrate the technical solutions in the embodiments of the present invention or the related technology, the accompanying drawings to be used in the description of the embodiments or the related technology will be briefly introduced below, and it will be obvious that the accompanying drawings in the following description are only some of the embodiments of the present invention, and that, for one skilled in the art, other accompanying drawings can be obtained based on these accompanying drawings without putting in creative labor.
FIG. 1 is a block diagram illustrating exemplary radiation treatment system according to some embodiments of the present invention.
FIG. 2 is a schematic diagram illustrating an exemplary radiation treatment system according to some embodiments of the present invention.
FIG. 3A is a schematic diagram illustrating an exemplary radiation beam emitter according to some embodiments of the present invention.
FIG. 3B is a schematic diagram illustrating another exemplary radiation beam emitter according to some embodiments of the present invention.
FIG. 4 is a schematic diagram illustrating another exemplary radiation treatment system according to some embodiments of the present invention.
FIG. 5 is a schematic diagram illustrating an exemplary radiation treatment system according to some embodiments of the present invention.
FIG. 6 is a schematic diagram illustrating an exemplary radiation treatment system according to some embodiments of the present invention.
FIG. 7 is a schematic diagram illustrating an exemplary radiation treatment system according to some embodiments of the present invention.
FIG. 8 is a flowchart illustrating an exemplary process for treating a target subject using a radiation treatment system according to some embodiments of the present invention.
FIG. 9 is a schematic diagram of an electron beam treatment device in an embodiment.
FIG. 10 is a schematic diagram of an electron beam treatment device in an embodiment.
FIG. 11 is a schematic diagram of an electron beam generator and an electron cyclotron in an embodiment.
FIG. 12 is a schematic diagram of a first deflection member and an annular deflection mechanism in an embodiment.
FIG. 13 is a schematic diagram of a second deflection member in an embodiment.
FIG. 14 is a schematic diagram of an annular deflection mechanism in an embodiment.
FIG. 15 is a schematic diagram of an electron beam treatment device in an embodiment.
FIG. 16 is a schematic diagram of an electron beam treatment device in an embodiment.
FIG. 17 is a schematic diagram of a system for flash therapy in an embodiment.

In the figures, 100, 200, 400, 500, 600, and 700 represent a radiation treatment system; 110 represents a radiation treatment device; 120 represents a movable supporting device; 130 represents an imaging device; 140 represents a processing device; 111 represents a radiation beam emitter; 112 represents a radiation beam adjustor; 130A represents an MRI scanner; 130B represents a liftable imaging device; 131, 141 represent a first main magnet; 132, 142 represent a second main magnet; 133 represents an accommodation space; 134 represents an open channel; 113 represents a beam regulator; 1121a and 1121b represent adjusting components; 1131 represents a beam splitting element; 1132 represents a constraint element; 1133 represents a deflection element; 1111 represents a radiation beam generator; 1112 represents a radiation beam accelerator; 1113 represents a guider; 1114 represents a deflector; 1112a represents an accelerating element; 1112b represents a direction control element; 150 represents a second radiation treatment device; 151 represents a second radiation beam emitter; 152 represents a second radiation beam adjustor; 160 represents a converter; 170 represents a collimator; 900 represents an electron beam treatment device; 9 represents an electron beam emitter; 10 represents an electron beam generator; 20 represents an electron cyclotron; 21 represents an acceleration cavity; 22 represents a vector magnet; 23 represents a lead-out magnet; 24 represents an auxiliary component; 30 represents a first deflection member; 40 represents an annular deflection mechanism; 50 represents a bed; and 60 represents a second deflection member.

### DETAILED DESCRIPTION OF THE EMBODIMENT

In the following detailed description, numerous specific details are set forth by way of examples in order to provide a thorough understanding of the relevant invention. However, it should be apparent to those skilled in the art that the present invention may be practiced without such details. In other instances, well-known methods, procedures, systems, components, and/or circuitry have been described at a relatively high level, without detail, in order to avoid unnecessarily obscuring aspects of the present invention. Various modifications to the disclosed embodiments will be readily apparent to those skilled in the art, and the general principles defined herein may be applied to other embodiments and applications without departing from the spirit and scope of the present invention. Thus, the present invention is not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

It will be understood that the term "system," "engine," "unit," "module," and/or "block" used herein are one method to distinguish different components, elements, parts, sections or assembly of different levels in ascending order. However, the terms may be displaced by another expression if they achieve the same purpose.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

It will be understood that, although the terms "first," "second," "third," "fourth," etc., may be used herein to describe various elements, these elements should not be limited by these terms. These terms are only used to distinguish one element from another. For example, a first element could be termed a second element, and, similarly, a second element could be termed a first element, without departing from the scope of example embodiments of the present invention.

Spatial and functional relationships between elements (for example, between crystal elements) are described using various terms, including "connected", "engaged", "interfaced", and "coupled". Unless explicitly described as being "direct", when a relationship between first and second elements is described in the present invention, that relationship includes a direct relationship where no other intervening elements are present between the first and second elements, and also an indirect relationship where one or more intervening elements are present (either spatially or functionally) between the first and second elements. In contrast, when an element is referred to as being "directly" connected, engaged, interfaced, or coupled to another element, there are no intervening elements present. Other words used to describe the relationship between elements should be interpreted in a like fashion (e.g., "between" versus "directly between", "adjacent" versus "directly adjacent", etc.). As used herein, the term "and/or" includes any and all combinations of one or more of the associated listed items.

An anatomical structure shown in an image of a subject (e.g., a patient) may correspond to an actual anatomical structure existing in or on the subject's body. The term "object" and "subject" in the present invention are used interchangeably to refer to a biological object (e.g., a patient, an animal) or a non-biological object (e.g., a phantom). In some embodiments, the object may include a specific part, organ, and/or tissue of the object. For example, the object may include the head, the bladder, the brain, the neck, the torso, a shoulder, an arm, the thorax, the heart, the stomach, a blood vessel, soft tissue, a knee, a foot, or the like, or any combination thereof, of a patient.

These and other features, and characteristics of the present invention, as well as the methods of operation and functions of the related elements of structure and the combination of parts and economies of manufacture, may become more apparent upon consideration of the following description with reference to the accompanying drawings, all of which form a part of this invention. It is to be expressly understood, however, that the drawings are for the purpose of illustration and description only and are not intended to limit the scope of the present invention. It is understood that the drawings are not to scale.

Conventionally, a radiation treatment system based on a radiation beam (e.g., an electron beam) usually uses a radiation beam emitter to emit the radiation beam, and the radiation beam emitter is mounted on a rotatable gantry that rotates around a rotation center. The radiation beam emitter rotates with the rotatable gantry so that a subject is irradiated by the radiation beam from multiple directions. However, the rotatable gantry is usually bulky and has a high cost (e.g., a high manufacturing cost, a maintenance cost, etc.), and the radiation treatment system has a low practicability.

An aspect of the present invention provides a radiation treatment system. The radiation treatment system may include a radiation treatment device and a movable supporting device. The radiation treatment device may be configured to emit a radiation beam towards a target region of a target subject for radiation treatment. The movable supporting device may be configured for supporting the target subject. The movable supporting device may be configured to adjust a position of the target region relative to the radiation treatment device during the radiation treatment such that the target region is irradiated by the radiation beam from different directions. For example, a radiation beam emitter of the radiation treatment device is configured to emit an initial radiation beam along a fixed direction, which may be adjusted by a radiation beam adjustor to form the radiation beam. The movable supporting device is a rotatable device that is separated from the radiation beam emitter of the radiation treatment device, and the target subject can set or stand on the rotatable device. When the rotatable device rotates, the target subject can be irradiated by the radiation beam from different directions.

Compared with the conventional radiation treatment system, the system of the present invention may achieve radiation treatment from different directions using the movable supporting device instead of the rotational gantry, which have a smaller size, a lower cost (e.g., a high manufacturing cost, a maintenance cost, etc.), thereby having a high practicability.

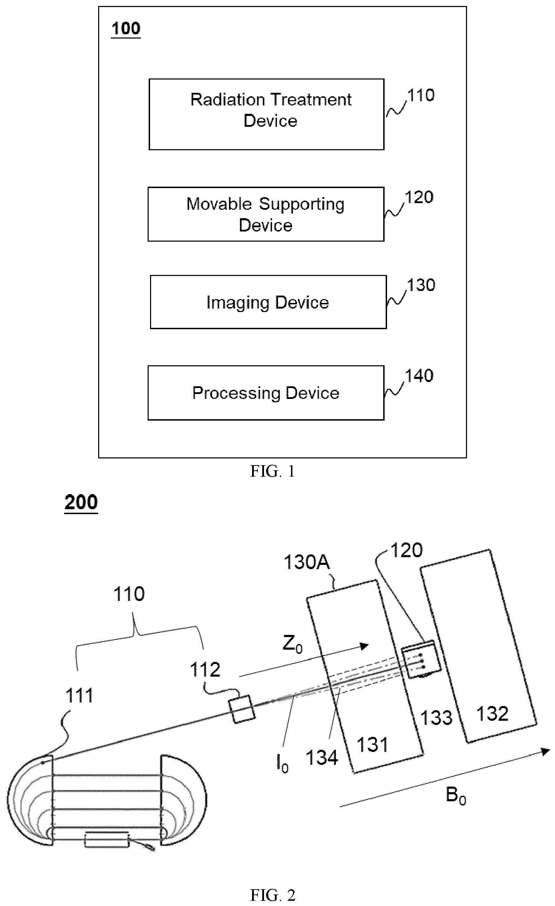

FIG. 1 is a block diagram illustrating exemplary radiation treatment system 100 according to some embodiments of the present invention.

In some embodiments, referring to FIG. 1, the radiation treatment system 100 may include a radiation treatment device 110, a movable supporting device 120, an imaging device 130, and a processing device 140. The components of the radiation treatment system 100 may be communicatively connected in various manners.

The radiation treatment device 110 may be configured to emit one or more radiation beams to a target region of a target subject for radiation treatment. The target subject may include a biological subject (e.g., a human being, an animal, a plant, or a portion thereof) and/or a non-biological subject (e.g., a phantom). A target region refers to a certain anatomical structure that needs to be tracked or monitored during the radiotherapy treatment. For example, the target region may be a tumor, an organ with a tumor, a tissue with a tumor, or any combination thereof, that needs to be treated by radiation. The radiation beam(s) may include an electron beam, a photon beam, a hadron (e.g., proton, ion) beam, X rays, a neutron beam, α rays, β rays, γ rays, or other types of radiation beam.

The movable supporting device 120 may be used for supporting the target subject. In some embodiments, the movable supporting device 120 may be configured to adjust a position of the target region relative to the radiation treatment device 110 during the radiation treatment such that the target region is irradiated by the radiation beam from different directions.

In some embodiments, the radiation treatment device 110 may include a radiation beam emitter configured to emit an initial radiation beam, and the initial radiation beam may be processed (e.g., split, shaped, deflected) to generate the radiation beam for irradiating the target region. In some embodiments, an irradiation direction of the radiation beam is substantially fixed. The irradiation direction of the radiation beam refers to a propagation direction of the radiation beam after the radiation beam leaves the radiation treatment device 110. Referring to FIG. 2, the irradiation direction of the radiation beam I₀ is a direction Z₀. The irradiation direction of the radiation beam towards the target region being substantially fixed may indicate that differences between irradiation directions of the radiation beams for different radiation treatments are below a threshold, e.g.,1%, 5%, or 10%.

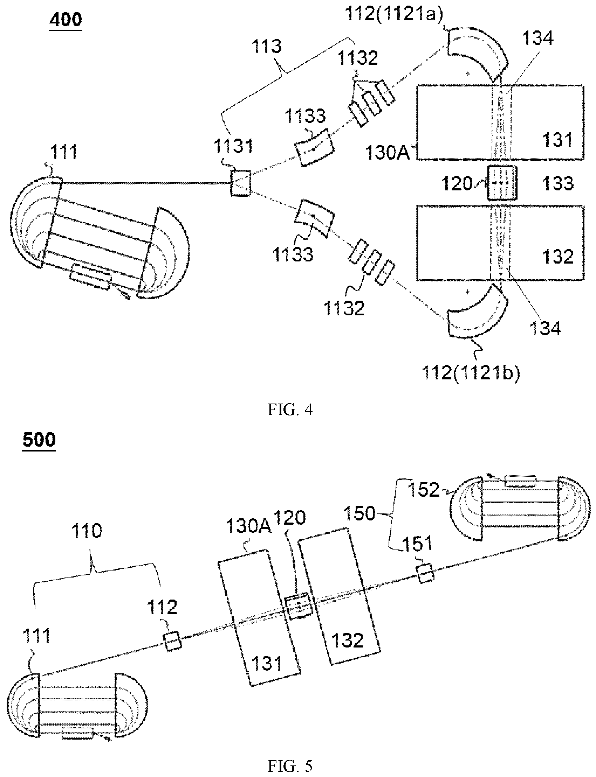

In some embodiments, the radiation beam emitter of the radiation treatment device 110 may be located at a fixed location for emitting the initial radiation beam along a fixed direction. The fixed direction refers to a moving direction of the initial radiation beam when the initial radiation beam leaves the radiation beam emitter (e.g., a direction pointing from the radiation beam emitter 111 towards the radiation beam adjustor 112 shown in FIG. 2, or a direction pointing from the radiation beam emitter 111 towards the beam regulator 113 shown in FIG. 4). In some embodiments, the radiation beam emitter may be positioned to the fixed location before the radiation treatment via a supporting component (e.g., a supporting frame). Alternatively, the supporting component is movable so that the radiation beam emitter can be positioned conveniently to different positions for performing different treatment.

As described elsewhere in the present invention, the radiation beam emitter of the conventional radiation treatment system is mounted on a rotatable gantry that rotates around a rotation center and rotates with the rotatable gantry. A subject to be treated and a supporting device for supporting the subject needs to be positioned within a rotation aperture formed by the rotation of the rotatable gantry to receive the radiation beam. Compared with the conventional radiation treatment system, since the radiation beam emitter of the radiation treatment device 110 is not mounted on a rotatable gantry, the movable supporting device 120 does not need to be positioned within a rotation aperture formed by the rotation of the rotatable gantry, the installation position of the movable supporting device 120 may be relatively flexible. Moreover, the installation positions of the radiation beam emitter can also be adjusted flexibly. In this way, the relative position between the radiation beam emitter and the movable supporting device 120 can be adjusted flexibly according to actual needs.

The imaging device 130 may generate or provide image data related to the target subject via scanning the target subject. For example, the imaging device 130 may obtain one or more images of the target region before, during, or after the radiation treatment. Specifically, the imaging device 130 may obtain images during the radiation treatment for achieving image guided radiation therapy (IGRT).

In some embodiments, the imaging device 130 may include a single modality imaging device and/or a multi-modality imaging device. The single modality imaging device may include, for example, an ultrasound imaging device, an X-ray imaging device, an computed tomography (CT) device, a magnetic resonance imaging (MRI) device, an ultrasonography device, a positron emission tomography (PET) device, an optical coherence tomography (OCT) imaging device, an ultrasound (US) imaging device, an intravascular ultrasound (IVUS) imaging device, a near-infrared spectroscopy (NIRS) imaging device, a far-infrared (FIR) imaging device, or the like, or any combination thereof. The multi-modality imaging device may include, for example, an X-ray imaging-magnetic resonance imaging (X-ray-MRI) device, a positron emission tomography-X-ray imaging (PET-X-ray) device, a single-photon emission computed tomography-magnetic resonance imaging (SPECT-MRI) device, a positron emission tomography-computed tomography (PET-CT) device, a C-arm device, a digital subtraction angiography-magnetic resonance imaging (DSA-MRI) device, etc.

In some embodiments, the imaging device 130 may be an MRI scanner located at a fixed location, and include an accommodation space for accommodating the target subject and the movable supporting device 120. The movable supporting device 120 may be located a position in the accommodation space. In some embodiments, the imaging device 130 may be a liftable computed tomography (CT) scanner or a liftable positron emission tomography (PET) scanner. The movable supporting device 120 may be located within an imaging channel of the liftable CT scanner or the liftable PET scanner.

The processing device 140 may process data obtained from the radiation treatment device 110, the imaging device 130, or other components of the radiation treatment system 100. In some embodiments, the processing device 140 may execute methods of the present invention. For example, the processing device 140 may cause radiation treatment to be delivered to a target subject according to a radiation treatment plan. Specifically, the processing device 140 may cause the radiation treatment device 110 to emit a radiation beam towards a target region of the target subject for radiation treatment according to the radiation treatment plan. The processing device 140 may cause the movable supporting device 120 to adjust a position of the target region relative to the radiation treatment device 110 according to the treatment plan such that the target region is irradiated by the radiation beam from different directions. Further, the processing device 140 may verify the radiation treatment plan in real-time during the radiation treatment based on one or more images collected by the imaging device 130 during the radiation treatment.

In some embodiments, the processing device 140 may be a single server or a server group. The server group may be centralized or distributed. In some embodiments, the processing device 140 may be local or remote. Merely for illustration, only one processing device 140 is described in the medical system 100. However, it should be noted that the medical system 100 in the present invention may also include multiple processing devices. Thus, operations and/or method steps that are performed by one processing device 140 as described in the present invention may also be jointly or separately performed by the multiple processing devices. For example, if in the present invention the processing device 140 of the medical system 100 executes both process A and process B, it should be understood that the process A and the process B may also be performed by two or more different processing devices jointly or separately in the medical system 100 (e.g., a first processing device executes process A and a second processing device executes process B, or the first and second processing devices jointly execute processes A and B).

It should be noted that the above description of the radiation treatment system 100 is merely provided for the purposes of illustration, and not intended to limit the scope of the present invention. For persons having ordinary skills in the art, multiple variations and modifications may be made under the teachings of the present invention. For example, the radiation treatment system 100 may include one or more additional components and/or one or more components of the radiation treatment system 100 described above may be omitted. For example, the radiation treatment system 100 may include a network configured to facilitate exchange of information and/or data. In some embodiments, one or more components (e.g., the radiation treatment device 110, the movable supporting device 120, the imaging device 130, or the processing device 140) of the radiation treatment system 100 may send information and/or data to other component(s) of the radiation treatment system 100 via the network. As another example, the radiation treatment system 100 may include a storage device configured to store data, instructions, and/or any other information. Additionally or alternatively, two or more components of the radiation treatment system 100 may be integrated into a single component. For example, the processing device 140 may be integrated into the radiation treatment device. A component of the radiation treatment system 100 may be implemented on two or more sub-components.

FIG. 2 is a schematic diagram illustrating an exemplary radiation treatment system 200 according to some embodiments of the present invention. In some embodiments, the radiation treatment system 200 may be an exemplary embodiment of the radiation treatment system 100 as described in connection with FIG. 1.

Referring to FIG. 2, the radiation treatment system 200 may include the radiation treatment device 110, the movable supporting device 120, an MRI scanner 130A, and the processing deceive (not shown).

The radiation treatment device 110 may be configured to emit a radiation beam towards a target region of a target subject for radiation treatment.

The radiation beam may have a certain energy level. In some embodiments, the radiation beam may have a high energy level. For example, the radiation beam is an electron beam, and an energy level of the electron beam may be greater than 50 megaelectron volts (MeV). Merely by way of example, the electron beam may have a high energy level in a range from 50 MeV to 300 MeV or 100 MeV to 300 MeV. In some embodiments, the radiation beam may have a low energy level. For example, the radiation beam is an electron beam with an energy lower than 50 MeV. In some embodiments, a radiation beam with a high energy level may be referred to as a high-energy radiation beam, and a radiation beam with a low energy level may be referred to as a low-energy radiation beam. The high-energy radiation beam may have better penetrability than the low energy radiation beam. The low energy radiation beam may be more suitable for treating a target on or near the body surface of the subject, while the high-energy radiation beam may be more suitable for treating a target inside the target subject.

The movable supporting device 120 may be used for supporting the target subject. The target subject may be supported by the movable supporting device 120 in various ways and need to maintain a specific posture during the radiation treatment. For example, the target subject may stand, lie, or sit on the movable supporting device 120. In some embodiments, the movable supporting device 120 may include a component (e.g., a backrest) for assisting the target subject to maintain a specific posture. In some embodiments, the movable supporting device 120 may include a chair, a table, a platform, or the like.

In some embodiments, the movable supporting device 120 may be configured to adjust a position of the target region relative to the radiation treatment device 110 during the radiation treatment such that the target region is irradiated by the radiation beam from different directions. In some embodiments, the movable supporting device 120 has multiple degrees of freedom of motion (6 degrees of freedom of motion) such that the position of the target region relative to the radiation treatment device 110 can be adjusted to a suitable position. In some embodiments, the movable supporting device 120 may be rotatable such that the target region is able to be irradiated by the radiation beam from any angle. Specifically, the movable supporting device 120 may be rotatable to change an orientation of the target region. For example, similar to aircraft movement, the movable supporting device 120 may perform a pitch operation, a roll operation, a yaw operation, etc., to rotate to ta appropriate position. Merly by way of example, when the target object is sitting on the movable supporting device 120 facing the radiation treatment device 110, the movable supporting device 120 may rotate so that the target object faces away from the radiation treatment device 110. In some embodiments, the movable supporting device 120 may be translatable such that different portions of the target region are able to be irradiated by the radiation beam. As another example, the movable supporting device 120 may move along different directions to change a position of the target region. For example, the movable supporting device 120 may go up or down. As another example, the movable supporting device 120 may move left or right.

In some embodiments, the radiation treatment device 110 may include a radiation beam emitter 111 and a radiation beam adjustor 112. The radiation beam adjustor 112 may be locate between the radiation beam emitter 111 and the movable supporting device 120.

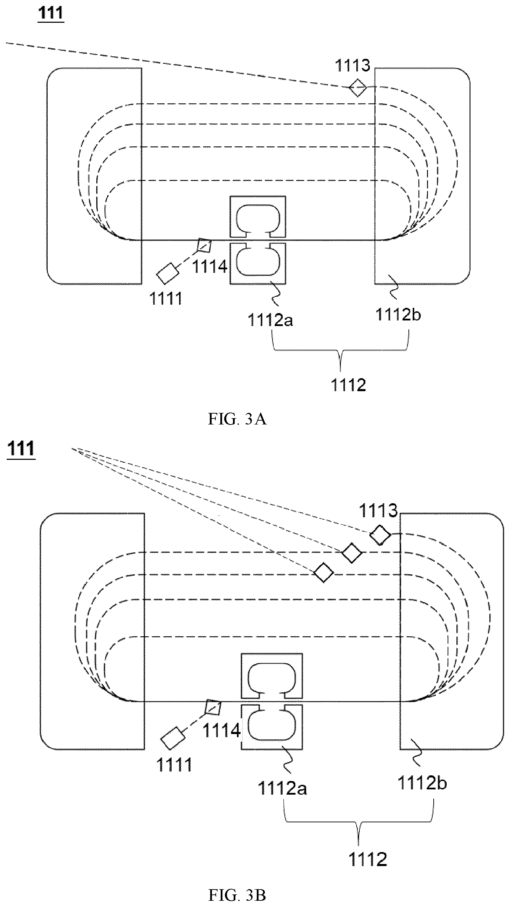

The radiation beam emitter 111 may be configured to emit an initial radiation beam. In some embodiments, the radiation beam emitter 111 may include a radiation beam generator, a radiation beam accelerator, and one or more guiders. The radiation beam emitter may be configured to generate the initial radiation beam. The radiation beam accelerator may be configured to accelerate the initial radiation beam. The one or more guiders may be configured to guide the accelerated initial radiation beam to enter into the radiation beam adjustor 112. More descriptions regarding the radiation beam emitter 111 may be found elsewhere in the present invention (e.g., FIGs. 3A and 3B, and the descriptions thereof).

The radiation beam adjustor 112 may be configured to adjust the initial radiation beam to generate the radiation beam that aligns to the target region. For example, if the radiation beam covers at least a portion of the target region, it is considered that the radiation beam aligns to the target region.

In some embodiments, the initial radiation beam generated by the radiation beam emitter 111 may be defocused, focused, shaped, deflected, etc., by the radiation beam adjustor 112 to generate the radiation beam and enter the target subject. In some embodiments, the radiation beam adjustor 112 may adjust the initial radiation beam to achieve a desired depth dose distribution in the target subject. As used herein, a depth refers to a distance with respect to the body surface of the target subject measured in a direction parallel ton a radiation beam that enters the target subject (e.g., a central axis of the radiation beam). A depth dose distribution refers to a distribution of radiation dose at different depths. A desired depth dose distribution may be achieved if a larger portion of the total radiation dose is deposited in the target region than in a non-target region (i.e., a region of the target subject other than the target region).

In some embodiments, the radiation beam adjustor 112 may include a deflection element and/or a beam profile modulator. The deflection element may be configured to adjust a moving trajectory (e.g., the moving direction) of the initial radiation beam, that is, the moving trajectory of the initial radiation beam may be altered by the deflection element when it passes through the deflection element. In some embodiments, the deflection element may include a scanning magnet that adjusts the initial radiation beam via a magnetic field, a microwave cavity that adjusts the initial radiation beam by applying a microwave field, an electric field applying device that adjusts the initial radiation beam by applying an electric field, or the like. The beam profile modulator may be configured to shape the initial radiation beam. Merely by way of example, the beam profile modulator may include one or more beam-limiting elements, such as a multi-leaf collimator, a blocker, that may block a specific portion of the initial radiation beam.

In some embodiments, the radiation beam emitter 111 may be separated from the radiation beam adjustor 112 as shown in FIG. 2. It should be noted that the positions of the radiation beam emitter 111 and the radiation beam adjustor 112 can be adjusted according to an actual need. For example, the radiation beam adjustor 112 may be mounted near an outlet (i.e., one or more exits of the one or more guiders) of the radiation beam emitter 111 for emitting the radiation beam.

The MRI scanner 130A may be an exemplary imaging device 130 as described in connection with FIG. 1, and a top view of the MRI scanner 130A is shown in FIG. 2. The MRI scanner 130A may be configured to acquire MRI images of the target region, which may be used to guide the radiation treatment in real-time or substantially real-time during the radiation treatment to compensate for motion (e.g., physiological motion, rigid body motion) of the target subject during the radiation treatment. An irradiation direction of the radiation beam may be parallel to (or substantially parallel to) a main magnetic field of the MRI scanner 130A to reduce or avoid the effect of the main magnetic field on the movement of the radiation beam. In some embodiments, a direction from the head to the feet of the target subject may be perpendicular to (or substantially perpendicular to) the main magnetic field B₀ of the MRI scanner 130A. Referring to FIG. 2, the irradiation direction Zo of the radiation beam I0 may be parallel to (or substantially parallel to) the main magnetic field B₀ of the MRI scanner 130A. The target subject may set or stand on the movable supporting device 120 and the direction from the head to the feet of the target subject may be perpendicular to (or substantially perpendicular to) the main magnetic field B₀ of the MRI scanner 130A.

The radiation beam may include multiple beamlets, and the irradiation direction of the radiation beam refers to an irradiation direction of the beamlet that passes through the center of the radiation beam. In some embodiments, the multiple beamlets of the radiation beam are substantially parallel. Merely by way of example, multiple beamlets are substantially parallel may indicate that differences between irradiation directions of the multiple beamlets are below a threshold, e.g.,1°, 2°.

Referring to FIG. 2, the MRI scanner 130Amay include a first main magnet 131, a second main magnet 132 that is disposed opposite to the first main magnet 131, and an accommodation space 133 between the first main magnet 131 and the second main magnet 132 for accommodating the target subject and the movable supporting device 120. At least one of the first main magnet 131 or the second main magnet 132 may include an open channel 134 for the radiation beam to pass through to irradiate the target region. For example, for the MRI scanner 130A as shown in FIG. 2, one of the first main magnet 131 and the second main magnet 132 that is close to the radiation treatment device 110 (i.e., the first main magnet 131) may include an open channel 134. As another example, for the MRI scanner as shown in FIG. 4, both the first main magnet 131 and the second main magnet 132 may include open channels 134.

In some embodiments, the first main magnet 141 and the second main magnet 142 may be independent. In some embodiments, the accommodation space 133 may be adjustable to facilitate the movement or rotation of the movable supporting device 120 and/or the movement of target objects into and out from the accommodation space 133. Alternatively, the first main magnet 141 and the second main magnet 142 may be connected via a connection element.

As described elsewhere in the present invention, the rotatable gantry of the conventional radiation treatment system is bulky and has a high cost, and the conventional radiation treatment system having a low practicability. Compared with the conventional radiation beam radiation treatment system, the radiation treatment system 200 may achieve radiation treatment from different directions using the movable supporting device 120 instead of using the rotatable gantry, which have a higher flexibility, a smaller size, a lower cost (e.g., a high manufacturing cost, a maintenance cost, etc.), thereby having a high practicability.

In some embodiments, the radiation treatment device 110 may further include a beam regulator configured to split the initial radiation beam into multiple initial sub-beams transmitting along different directions, or switch (or change) the irradiation direction of the initial radiation beam to a desired direction. Correspondingly, in some embodiments, when the beam splitting regulator is configured to switch (or change) the irradiation direction of the initial radiation beam to a desired direction, the radiation beam adjustor 112 may include multiple adjusting components each of which is configured to adjust a switched initial beam to generate the radiation beam corresponding to a desired direction. In some embodiments, when the beam splitting regulator is configured to split the initial radiation beam into multiple initial sub-beams transmitting along different directions, the radiation beam adjustor 112 may include multiple adjusting components configured to adjust the initial sub-beams, respectively, to generate multiple sub-beams of the radiation beam. In some embodiments, an initial sub-beam may be defocused, focused, shaped, change an irradiation direction, etc., by one of the multiple adjusting components to generate the corresponding sub-beam and enter the target subject. For example, the adjustment component may adjust the irradiation direction of the initial sub-beam so that the generated sub-beam irradiates towards the target region. In some embodiments, the multiple adjusting components may be of the same type or different types. The multiple sub-beams may irradiate the target region from different directions. In some embodiments, energy levels or shapes of the multiple sub-beams may be the same or different. If the imaging device is an MRI scanner, the irradiation directions of the multiple sub-beams may be parallel to the main magnetic field of the MRI scanner.

For example, FIG. 4 is a schematic diagram illustrating another exemplary radiation treatment system 400 according to some embodiments of the present invention. The difference between the radiation treatment system 400 and the radiation treatment system 200 shown in FIG. 2 is that the radiation treatment device of the radiation treatment system 400 further include a beam regulator 113 configured to split the initial radiation beam into two initial sub-beams transmitting along different directions. The radiation beam adjustor 112 includes adjusting components 1121a and 1121b configured to adjust the two initial sub-beams, respectively, to generate two sub-beams of the radiation beam. The two sub-beams may irradiate the target region from different directions.

In some embodiments, the beam regulator 113 may include a beam splitting element 1131 and multiple constraint elements 1132. The beam splitting element 1131 may be configured to split the initial radiation beam into the multiple initial sub-beams. In some embodiments, the beam splitting element 1131 may include a magnetic switch. The multiple constraint elements 1132 may be configured to constrain transmission directions of the multiple initial sub-beams so that each of the multiple initial sub-beams enters into one of the multiple adjusting components 1121. In some embodiments, a constraint element 1132 may include a four-pole magnetic switch.

In some embodiments, the beam regulator 113 may include multiple deflection elements 1133. A deflection element 1133 may be configured to guide an initial sub-beam split by the beam splitting element 1131 into one constraint element 1132 by adjusting a moving direction of the initial sub-beam.

According to some embodiments of the present invention, by using the beam regulator 113, radiation treatment can be delivered to the target region from multiple directions simultaneously, thereby improving the efficiency of the radiation treatment.

FIG. 3A is a schematic diagram illustrating an exemplary radiation beam emitter 111 according to some embodiments of the present invention. Referring to FIG. 3A, the radiation beam emitter 111 may include a radiation beam generator 1111, a radiation beam accelerator 1112, and one or more guiders 1113. The radiation beam emitter 1111 may be configured to generate the initial radiation beam. The radiation beam accelerator 1112 may be configured to accelerate the initial radiation beam. The one or more guiders 1113 may be configured to guide the accelerated initial radiation beam to enter into the radiation beam adjustor 112. In some embodiments, the radiation beam emitter 111 may further include a deflector 1114. The deflector 1114 may be configured to guide the initial radiation beam generated by the radiation beam emitter 1111 into the radiation beam accelerator 1112 by adjusting a moving direction of the initial radiation beam.

In some embodiments, the radiation beam accelerator 1112 may include an accelerating element 1112a and a direction control element 1112b. The accelerating element 1112a may be configured to accelerate the initial radiation beam. In some embodiments, the accelerating element 1112a may be a resonant cavity (e.g., a waveguide resonant cavity and a coaxial linear resonant cavity). A volume of the waveguide resonant cavity may be larger than that of the coaxial linear resonant cavity. The coaxial linear resonant cavity may include multiple parallel cavities, and a number of the cavities may be set according to needs, such as the increased energy required for each acceleration.

The direction control element 1112b may be configured to control a moving direction of the initial radiation beam so that the initial radiation beam returns to the accelerating element 1112a one or more times. In some embodiments, referring to FIG. 3A, the direction control elements 1112b may include two parts that are symmetrically arranged on both sides of the accelerating element 1112a to guide the initial radiation beam returns to the accelerating element 1112a one or more times. For example, the initial radiation beam may return into the accelerating element 1112a one or more times along one or more annular trajectories (e.g., circular dashed trajectories shown in FIG. 3A). That is, the initial radiation beam may continuously pass through the accelerating element 1112a, each time the initial radiation beam returns into the accelerating element 1112a, it may be accelerated again and its energy level may be increased, and the movement trajectory may be longer than the last movement trajectory. In some embodiments, the direction control element 1112b may include one or more deflection magnets that can deflect the initial radiation beam.

The one or more guiders 1113 may be located at one or more annular trajectories according to desired energy levels. After a certain time of acceleration, the energy level of the initial radiation beam may reach a desired level and the initial radiation beam having the desired energy level may be guided out by the one or more guiders 1113.

In some embodiments, the radiation beam accelerator 1112 may include an electron cyclotron. Exemplary electron cyclotron may be or include a betatron, a petal-shaped accelerator, a race-track microtron, or the like. The electron cyclotron may have a relatively small size and a low manufacturing cost, thereby reducing the size and the manufacturing cost of the radiation treatment device 110.

In some embodiments, the initial radiation beam may return into the accelerating element 1112a multiple times along multiple annular trajectories. The one or more guiders 1113 include multiple guiders 1113 disposed on different annular trajectories of the multiple annular trajectories for guiding radiation beams with different energy levels. For example, FIG. 3B is a schematic diagram illustrating another exemplary radiation beam emitter 111 according to some embodiments of the present invention. Referring to FIG. 3B, three guiders 1113 are disposed on three annular trajectories for guiding radiation beams with different energy levels. In some embodiments, the one or more guiders 1113 include one or more movable guiders that are capable of moving to different annular trajectories of the multiple annular trajectories for guiding radiation beams with different energy levels. For example, the radiation beam emitter 111 is provided with one or more installation channels, and the one or more movable guiders 1113 are arranged in the one or more installation channels. Positions of the one or more movable guiders 1113 may be adjusted by a control component (e.g., a telescopic cylinder or a telescopic motor).

In operation, one of the three guiders 1113 may be actuated to guide the radiation beam with a desired level, or two or all of the guiders 1113 may be actuated alternately to guide radiation beams with different energy levels alternately when a multi-energy level radiation beam radiation treatment is performed. In this way, radiation beams with different energy levels may be easily obtained, so that different treatment needs can be satisfied and the accuracy of radiation treatment can be improved.

In some embodiments, the radiation beam emitter 111 may further include an auxiliary component (e.g., an electromagnet). The auxiliary component may be configured to adjust the movement trajectory of the initial radiation beam and/or assist in accelerating the initial radiation beam, etc.

FIG. 5 is a schematic diagram illustrating an exemplary radiation treatment system 500 according to some embodiments of the present invention. In some embodiments, the radiation treatment system 500 may be an exemplary embodiment of the radiation treatment system 100 as described in connection with FIG. 1.

Referring to FIG. 5, the radiation treatment system 500 may be similar to the radiation treatment system 200 as described in connection with FIG. 2, except that the radiation treatment system 500 may further include a second radiation treatment device 150.

The second radiation treatment device 150 may be configured to emit a second radiation beam towards the target region of the target subject for the radiation treatment. In some embodiments, the second radiation treatment device 150 may be the same as or similar to the radiation treatment device 110 (also referred to as the first radiation treatment device 110). The second radiation treatment device 150 may include a second radiation beam emitter 151 and a second radiation beam adjustor 152. The radiation beam (also referred to as the first radiation beam) emitted by the first radiation treatment device 110 and the second radiation beam may irradiate the target region from different directions.

In some embodiments, positions of the first radiation treatment device 110 and the second radiation treatment device 150 may be set according to a type of the imaging device 130, a position of the movable supporting device 120, etc. For example, if the imaging device 130 is an MRI scanner 130A, the irradiation directions of the first radiation beam and the second radiation beam may be parallel to a main magnetic field of the MRI scanner. As another example, the first radiation treatment device 110 and the second radiation treatment device 150 may be symmetrically arranged relative to the movable supporting device 120.

In some embodiments, energy levels of the first radiation beam and the second radiation beam may be determined based on the position of the target region relative to the first radiation treatment device 110 and the second radiation treatment device 150. For example, if the target region is farther from the first radiation treatment device 110 than from the second radiation treatment device 150, the energy level of the first radiation beam may be greater than the energy level of the second radiation beam. If the target region is closer to the first radiation treatment device 110 than to the second radiation treatment device 150, the energy level of the first radiation beam may be smaller than the energy level of the second radiation beam.

In some embodiments, the position of the target region relative to the first radiation treatment device 110 and the second radiation treatment device 150 may be determined based on an image of the target region captured by an imaging device (e.g., the MRI scanner 130A) during the radiation treatment. Specifically, positions of the target subject, the first radiation treatment device 110, and the second radiation treatment device 150 may be obtained. For example, the positions of the target subject, the first radiation treatment device 110, and the second radiation treatment device 150 may be determined based on an optical image captured by an image acquisition device (e.g., a camera). As another example, the positions of the target subject, the first radiation treatment device 110, and the second radiation treatment device 150 may be previously determined. A position of the target region in the target subject may be determined based on the image of the target region. Further, the position of the target region relative to the first radiation treatment device 110 and the second radiation treatment device 150 may be determined based on the position of the target region in the target subject, the positions of the target subject, the first radiation treatment device 110, and the second radiation treatment device.

The radiation treatment system 500 may use both the first radiation treatment device 110 and the second radiation treatment device simultaneously to perform the radiation treatment on the target subject, which may improve the efficiency of the radiation treatment. In addition, in some embodiments, the energy levels of the first radiation beam and the second radiation beam may be determined based on the position of the target region relative to the first radiation treatment device 110 and the second radiation treatment device 150, which may improve the accuracy of the radiation treatment.

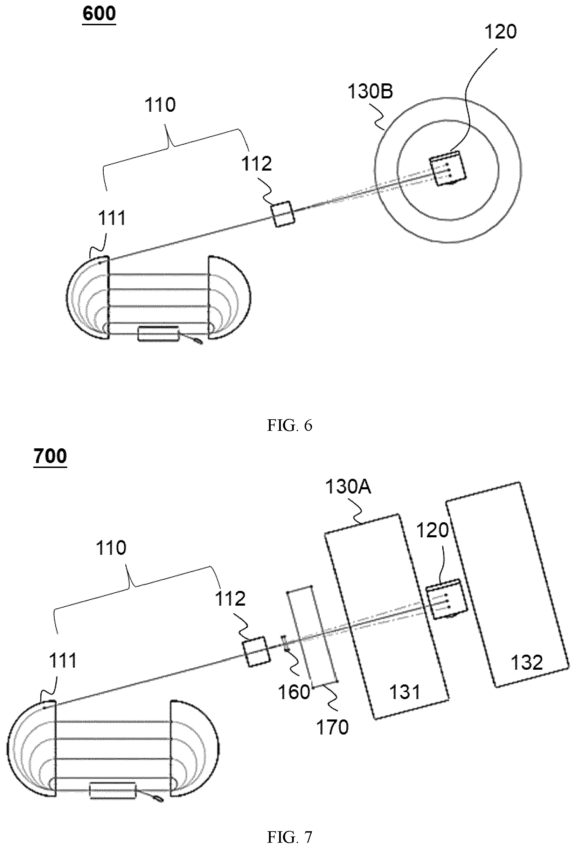

FIG. 6 is a schematic diagram illustrating an exemplary radiation treatment system 600 according to some embodiments of the present invention. In some embodiments, the radiation treatment system 600 may be an exemplary embodiment of the radiation treatment system 100 as described in connection with FIG. 1.

Referring to FIG. 6, the radiation treatment system 600 may be similar to the radiation treatment system 200 as described in connection with FIG. 2, except that the radiation treatment system 600 includes a liftable imaging device 130B different from the MRI scanner 130A of the radiation treatment system 200. The liftable imaging device 130B may include a liftable computed tomography (CT) scanner or a liftable positron emission tomography (PET) scanner.

The liftable imaging device 130B may include a gantry that can be lifted up or put down. Other components of the liftable imaging device 130B (e.g., an X-ray source, detectors) may be lifted up or put down with the gantry. For example, the gantry may be suspended from a ceiling of a room where the radiation treatment system 600 is located via a retractable suspension device, and can be lifted up or put down by the retractable suspension device. Referring to FIG. 6, in some embodiments, the gantry of the liftable imaging device 130B has a circular structure, and the movable supporting device 120 is located within an imaging channel of the gantry.

The gantry of the liftable imaging device 130B may be lifted up when the radiation beam is emitted by the radiation treatment device 110 to avoid blocking the radiation beam. The gantry of the liftable imaging device 130B may be put down and surround the target subject for obtaining the image when the radiation beam is stopped. For example, the radiation beam may be delivered to the target subject multiple times during the radiation treatment. Each time the radiation beam is delivered for a certain period of time, the radiation beam may be stopped and the gantry of the liftable imaging device 130B may be put down and surround the target subject for obtaining the image of the target region. Then, a radiation treatment plan of the radiation treatment may be verified based on the image. Further, the gantry of the liftable imaging device 130B may be lifted up, and then the radiation beam may be delivered to the target subject again according to radiation treatment plan or an updated radiation treatment plan.

FIG. 7 is a schematic diagram illustrating an exemplary radiation treatment system 700 according to some embodiments of the present invention. In some embodiments, the radiation treatment system 700 may be an exemplary embodiment of the radiation treatment system 100 as described in connection with FIG. 1.

Referring to FIG. 7, the radiation treatment system 700 may be similar to the radiation treatment system 200 as described in connection with FIG. 2, except that the radiation treatment system 700 further comprises a converter 160 and a collimator 170 disposed between the radiation treatment device 110 and the movable supporting device 120 (or the MRI scanner 130A).

The converter 160 may be configured to convert the first radiation beam into a third radiation beam (e.g., a photon beam). The collimator 170 may be configured to adjust the third radiation beam to align to the target region. For example, the collimator 170 may adjust an intensity, shape, etc., of the third radiation beam. The collimator 170 may be a beam-limiting device, such as a multi-leaf collimator, a blocker, that may block a specific portion of the third radiation beam. The specific portion of the third radiation beam may cause dose disposition in the non-target region of the target subject if it is not blocked. In some embodiments, the shape of the collimator 170 may be adjusted such that the remaining portion of the third radiation beam may be better aligned to the target region.

In some embodiments, the converter 160 and the collimator 170 may be mounted on the radiation treatment device 110. Alternatively, the converter 160 and the collimator 170 may be mount on another supporting component and be separated from the radiation treatment device 110. In some embodiments, the converter 160 and the collimator 170 may be movable or detachable such that the radiation treatment system 700 may perform the radiation treatment in a similar manner as the radiation treatment system 200. In this way, the radiation treatment system 700 can be used to deliver the third radiation beam and/or the radiation beam for radiation treatments.

It should be noted that systems and devices illustrated in FIG. 7 and the descriptions thereof are provided for the purposes of illustration, and not intended to limit the scope of the present invention. For persons having ordinary skills in the art, various modifications and changes in the forms and details of the application of the above method and system may occur without departing from the principles of the present invention. However, those variations and modifications also fall within the scope of the present invention. For example, the MRI scanner 130A of the radiation treatment systems 200, 400, 500, 700, and the liftable imaging device 130B of the radiation treatment system 600 may be replaced by another imaging device. As another example, the count of radiation treatment devices may be more than two.

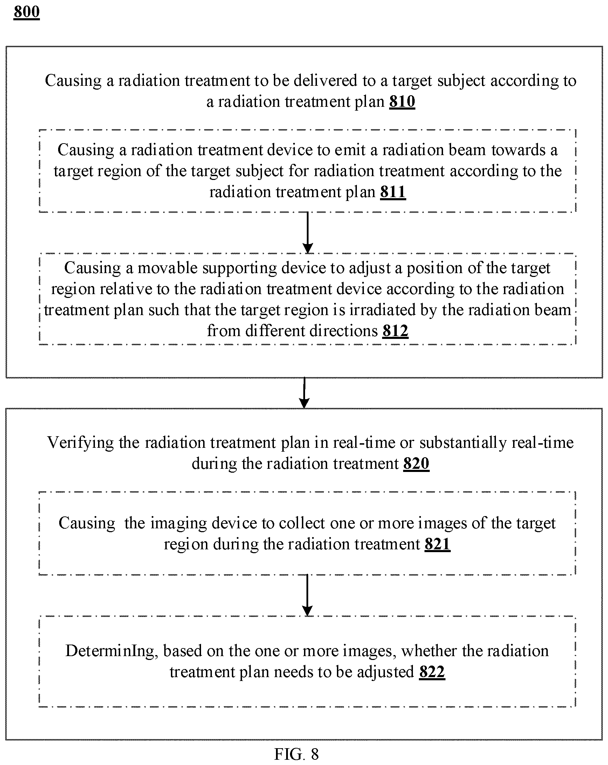

FIG. 8 is a flowchart illustrating an exemplary process 800 for treating a target subject using a radiation treatment system according to some embodiments of the present invention. The radiation treatment system may be any one of the radiation treatment systems 200, 400, 500, 600, and 700 disclosed herein. In some embodiments, the process 800 may be stored in a storage as a form of instructions, and invoked and/or executed by the processing device 140. The operations of the process 800 are intended to be illustrative. In some embodiments, the process 800 may be accomplished with one or more additional operations not described, and/or without one or more of the operations discussed. Additionally, the order in which the operations of the process 800 is not intended to be limiting.

In 810, the processing device 140 may cause a radiation treatment to be delivered to a target subject according to a radiation treatment plan.

Specifically, the radiation treatment plan is previously generated based on a planning image (e.g., a planning CT image) of the target subject and describes how the radiation treatment is going to be performed.

As described above, the movable supporting device 120 of the radiation treatment system may rotate so that the target subject is irradiated by a radiation beam emitted by the radiation treatment device 110 from multiple directions. The radiation treatment plan may include treatment parameters corresponding to each of the directions. In some embodiments, the treatment parameters corresponding to a specific direction may include planned motion parameters of the movable supporting device 120 and planned beam parameters of the radiation beam corresponding to the direction. The planned motion parameters of the movable supporting device 120 may specify how the movable supporting device 120 moves so that the target subject can be irradiated from the specific directions. Exemplary planned motion parameters of the movable supporting device 120 may include a rotation time (which specifies when the movable supporting device 120 rotates during the radiation treatment), a ration angle (which specifies the rotation amplitude of the movable supporting device 120), or the like, or any combination thereof. The planned beam parameters may specify what the radiation beam is used to irradiate the target subject under the specific direction. Exemplary planned beam parameters of the radiation beam may include an energy level, a shape, an irradiation direction, an irradiation time, or the like, or any combination thereof.

Referring to FIG. 8, in some embodiments, operation 810 may include operations 811 and 812. In 811, the processing device 140 may cause the radiation treatment device 110 to emit the radiation beam towards the target region of the target subject for radiation treatment according to the radiation treatment plan. In 812, the processing device 140 may cause the movable supporting device 120 to adjust a position of the target region relative to the radiation treatment device 110 according to the radiation treatment plan such that the target region is irradiated by the radiation beam from different directions.

For example, for each direction, the processing device 140 determine the treatment parameters corresponding to the direction based on the radiation treatment plan. The processing device 140 may cause the movable supporting device 120 to adjust the position of the target region relative to the radiation treatment device 110 according to the planned motion parameters of the movable supporting device 120 corresponding to the direction. Further, the processing device 140 may cause the radiation treatment device 110 to emit the radiation beam towards the target region according to the planned beam parameters of the radiation beam corresponding to the direction such that the target region is irradiated by the radiation beam from the direction.

In 820, the processing device 140 may verify the radiation treatment plan in real-time or substantially real-time during the radiation treatment. For example, the verification of the radiation treatment plan may be used to compensate for motion (e.g., physiological motion, rigid body motion) of the target subject during the radiation treatment. Specifically, operation 820 may include operations 821 and 822 as shown in FIG. 8. In 821, the processing device 140 may cause the imaging device 130 to collect one or more images of the target region during the radiation treatment. In 822, the processing device 140 may determine whether the radiation treatment plan needs to be adjusted based on the one or more images.

For example, each time the imaging device 130 collect a current image. The processing device 140 may determine a current feature of the target region based on the current image of the target region. Exemplary features of the target region may include a position, a shape, a size, etc., of the target region. The processing device 140 may determine whether a difference between the current feature of the target region and a historical feature of the target region in a historical image exceeds a preset threshold. The historical image may be the last image collected by the imaging device 130 or the previous image collected by the imaging device 130 when the radiation beam is adjusted last time. For example, the processing device 140 may determine whether a size difference between the current size of the target region and a historical size of the target region in the historical image exceeds a size threshold. In response to determining that the difference exceeds the preset threshold (e.g., the size difference exceeds the size threshold), the processing device 140 may determine that the radiation treatment plan needs to be adjusted, and adjust the radiation treatment plan such that the current target region can be targeted by the radiation beam. For example, the planned beam parameters (e.g., the irradiation direction, the shape, the energy level) of the radiation beam in the radiation treatment plan may be adjusted based on the current feature of the target region. As another example, the planned motion parameters of the movable supporting device 120 may be adjusted based on the current feature of the target region.

After the radiation treatment plan is adjusted, the radiation treatment may be delivered according to the adjusted radiation treatment plan. For example, the radiation beam may be emitted according to the adjusted beam parameters and/or the movable supporting device 120 may move according to the adjusted motion parameters. The real-time adjustment of the treatment plan may improve the aligning of the radiation beam to the current target region to improve the treatment accuracy.

In another aspect, the above radiation treatment system can be applied to electron beam treatment. An electron beam treatment device 900 is provided, which is configured to generate and deliver a high-energy electron beam that is applicable to flash therapy. Energy of the high-energy electron beam may be higher than 50 MeV, such as in a range of 100MeV to 200MeV. When the energy of the high-energy electron beam is higher than 50MeV, a maximum penetration depth and a lateral half-shadow quality in a radiotherapy application may be greatly improved. Because the energy of the high-energy electron beam is far greater than that of a common high-energy electron beam, in some cases, the high-energy electron beam may also be referred to as an "ultra-high-energy electron beam". The high-energy electron beam generated by the electron beam treatment device 900 in the present invention may implement radiation transmission with ultra-high dosage rate (for example, 40Gy/s or even 100Gy/s). Therefore, an expected radiotherapy may be completed in a very short time. In addition, when a tumor of a patient is irradiated with a high-energy electron beam, a requirement for using deep therapy may be implemented.

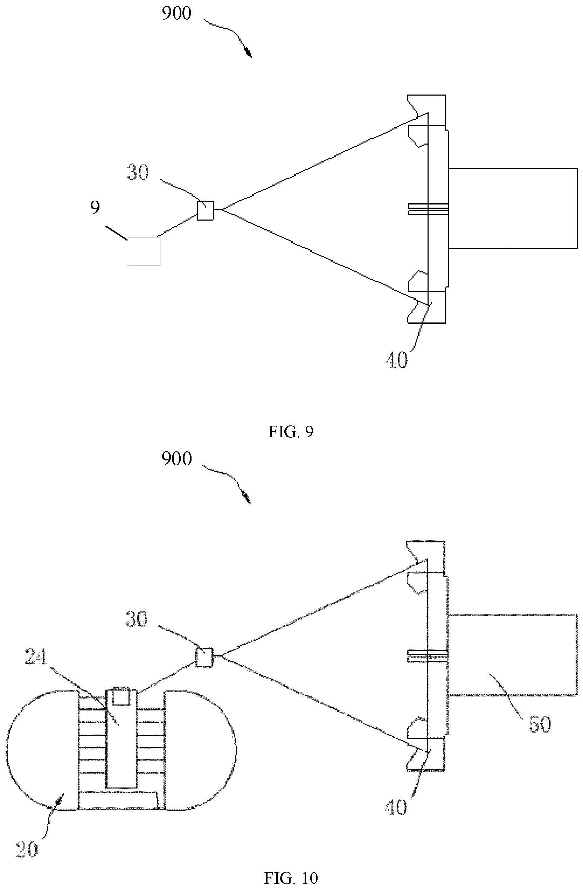

Referring to FIG. 9, an electron beam treatment device 900 provided in an embodiment of the present invention includes an electron beam emitter 9, a first deflection member 30, and an annular deflection mechanism 40. The electron beam emitter is configured to emit a high-energy electron beam. The first deflection member 30 is capable of performing first deflection processing on the high-energy electron beam. The annular deflection mechanism 40 is configured to receive the high-energy electron beam deflected by the first deflection member 30, and perform second deflection processing on the high-energy electron beam, so that the high-energy electron beam is emitted towards a target region. Alternatively, the first deflection member 30 may be disposed on a path of the high energy electron beam.

In an embodiment, the electron beam emitter 9 may include an electron beam generator 10 and an electron cyclotron 20, the electron beam generator 10 is configured to generate an electron beam, and the electron cyclotron 20 is configured to receive an electron beam from the electron beam generator, and perform cyclotron acceleration processing on the electron beam, so that the electron beam is formed into the high-energy electron beam.

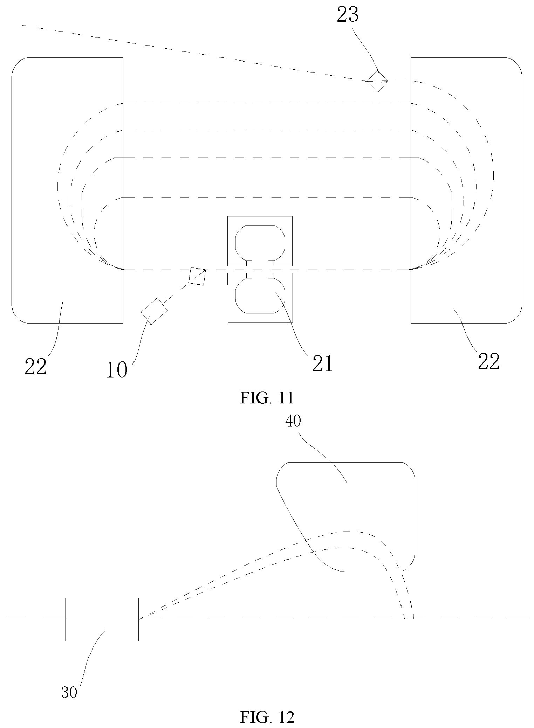

Referring to FIG. 10 and FIG. 11, an electron beam treatment device 900 provided in an embodiment of the present invention includes an electron beam generator 10, an electron cyclotron 20, a first deflection member 30, and an annular deflection mechanism 40. The electron beam emitter 9 may include the electron beam generator 10 and the electron cyclotron 20. The electron beam generator 10 is configured to generate an electron beam. The electron cyclotron 20 is configured to receive the electron beam from the electron beam generator 10, and perform cyclotron acceleration processing on the electron beam, so that the electron beam is formed into a high energy electron beam. The first deflection member 30 is disposed on a path of the high-energy electron beam, and the first deflection member 30 is capable of performing first deflection processing on the high-energy electron beam. The annular deflection mechanism 40 is configured to receive the high-energy electron beam deflected by the first deflection member 30, and perform second deflection processing on the high-energy electron beam, so that the high-energy electron beam is emitted towards a target region.

It may be understood that acceleration of the electron beam is implemented in a cyclotron acceleration manner, so that continuous multi-circle acceleration processing may be performed on the electron beam when the electron cyclotron 20 is in operation. In this way, a volume required when the electron cyclotron 20 is to accelerate the electron beam to a high-energy electron beam that meets a flash treatment requirement may be reduced, and acceleration efficiency of the electron beam may be improved, thereby reducing a volume of the electron beam treatment device 900 and reducing costs. In addition, the electron beam treatment device 900 may control intensity of an electromagnetic field generated when the first deflection member 30 is in operation, so as to implement final target region control of high-energy electron beam aggregation or delivery. In this way, efficiency and sensitivity of adjusting the target region are improved after the high-energy electron beam is converged when the electron beam treatment device 900 is in operation.

It should be noted that the target region refers to a location at which treatment is expected to be performed. As a non-limiting example, the target region may be an isocenter region, a specific volume around the isocenter region, or several discrete point positions. As a specific example, the target region may include a region of interest (ROI), for example, a location at which a tumor or another lesion is located in a patient to which the electron beam treatment device 900 functions when the electron beam treatment device 900 operates, that is, the electron beam treatment device 900 may accurately irradiate the tumor or the lesion when the electron beam treatment device 900 operates, and achieve a purpose of the flash therapy. The first deflection member 30 is capable of performing, by an electron field, a magnetic field, or a combination thereof, first deflection processing on the high-energy electron beam passing through the first deflection member 30. The magnetic field may be a permanent magnetic field, an electromagnetic field, or a combination thereof.

In some embodiments, the electron beam generator 10 is specifically configured as an electron gun, and when the electron gun is in operation, the electron beam may be emitted to the electron cyclotron 20. It may be understood that the electron beam generator 10 is not limited to the electron gun. For one skilled in the art, the electron beam generator 10 may be configured as another apparatus that can generate the electron beam, for example, an electron transmitter, an electron diode, and so on.

Referring to FIG. 11, the electron cyclotron 20 may include an acceleration cavity 21 and two groups of vector magnets 22, and the acceleration cavity 21 is capable of performing acceleration processing on the electron beam in the acceleration cavity 21. The two groups of vector magnets 22 may be symmetrically disposed at both sides of the acceleration cavity 21 and configured to guide the electron beam emitted from the acceleration cavity 21 to re-enter the acceleration cavity 21. That is, when the electron cyclotron 20 is in operation, the electron beam may circulate in the electron cyclotron 20 and pass through the acceleration cavity 21 continuously, and acceleration of the electron beam may be performed by the acceleration cavity 21, so that a circle formed by each circulation path of the electron beam gradually increases. At the same time, energy of the electron beam may be also continuously strengthened. It should be noted that, when the two groups of vector magnets 22 are in operation, deflection processing may be performed on the electron beam, so as to drive the electron beam to turn. It should be noted that the high-energy electron beam formed when the electron cyclotron 20 is in operation has a relatively high beam current, so that the high-energy electron beam meets a requirement of radiation dosage rate of a flash therapy.

The acceleration cavity 21 may be configured as a waveguide resonant cavity or a coaxial linear resonant cavity, so that each time the electron beam passes through the waveguide resonant cavity or the coaxial linear resonant cavity, a specific energy gain may be obtained, and an objective of accelerating the electron beam may be achieved.

The electron cyclotron 20 may further include a lead-out magnet 23. The lead-out magnet 23 may be disposed on a cyclic path of the electron beam in the electron cyclotron 20, and may be configured to lead out the high-energy electron beam formed after acceleration from the accelerating cavity 21. It should be noted that a specific structure of the lead-out magnet 23 and an operation principle of how to implement lead-out of the high-energy electron beam is described in a conventional form, which is not described herein.

In some embodiments, a position of the lead-out magnet 23 is capable of being adjusted relative to the electron cyclotron 20.

It may be understood that different positions of the lead-out magnet 23 in the electron cyclotron 20 are in a one-to-one correspondence with different cyclic paths of the electron beam in the electron cyclotron 20. In this way, different energy choices for the electron cyclotron 20 to derive the electron beam from the lead-out magnet 23 may be implemented by adjusting the position of the lead-out magnet 23 in the electron cyclotron 20. In this way, it is convenient for the electron cyclotron 20 to control selection of electron beams with different energy, and meet different requirements of the electron beam treatment device 900 when in operation.

Specifically, a mounting channel (not shown in the figures) may be disposed on the electron cyclotron 20. The lead-out magnet 23 may be specifically disposed in the mounting channel, and may be controlled by a telescopic cylinder or a telescopic motor to adjust positions of the lead-out magnet 23 in the mounting channel. In this way, control may be implemented for adjusting different positions of the lead-out magnet 23 in the mounting channel, that is, automatic control may be implemented to adjust the position of the lead-out magnet 23 in the electron cyclotron 20.

In some embodiments, the electron cyclotron 20 may include at least one of a race-track microtron, a betatron, or a petal-shaped accelerator.

In addition, alternatively, referring to FIG. 10, the electron cyclotron 20 may further include an auxiliary component 24. The auxiliary component 24 may be configured to adjust a path of an accelerated electron and/or perform auxiliary acceleration on the electron. As a non-limiting example, the auxiliary component 24 may be an electromagnet.

It may be learned from the foregoing that the first deflection member 30 can generate an electromagnetic field, and perform first deflection processing on the high-energy electron beam passing through the electromagnetic field by the electromagnetic field. Therefore, referring to FIG. 12, when the electron beam treatment device 900 is in operation, a generated magnetic field strength is capable of being adjusted when the first deflection member 30 is powered on, so as to adjust an angle at which the high-energy electron beam passing through the first deflection member 30 enters the annular deflection mechanism 40. In this way, an objective of changing the target region may be achieved, and a requirement for irradiating a region of interest such as a tumor is met. The high-energy electron beam passing through the first deflection member 30 is capable of entering the annular deflection mechanism 40 at different angles, which means that the high-energy electron beam passing through the first deflection member 30 may enter different circumferential positions of the annular deflection mechanism 40, and/or enter different radial positions of the annular deflection mechanism 40.

It may be understood that, when the electron beam treatment device 900 is in operation, a current of the first deflection member 30 when powered on may be adjusted to implement adjustment of an electromagnetic strength of the first deflection member 30 when powered on. In this way, it may facilitate adjustment of the target region at which the high-energy electron beam is converged when the electron beam treatment device 900 is in operation. The high-energy electron beam may be changed by the first deflection member 30 to adjust the target region, such as axial electron beam scanning.

In some embodiments, the first deflection member 30 may include either or both of a vector magnet and a deflection resonant cavity. When the first deflection member 30 is configured as a vector magnet, the vector magnet may be alternatively configured as a group of vector magnets that are orthogonal and have adjustable deflection directions.

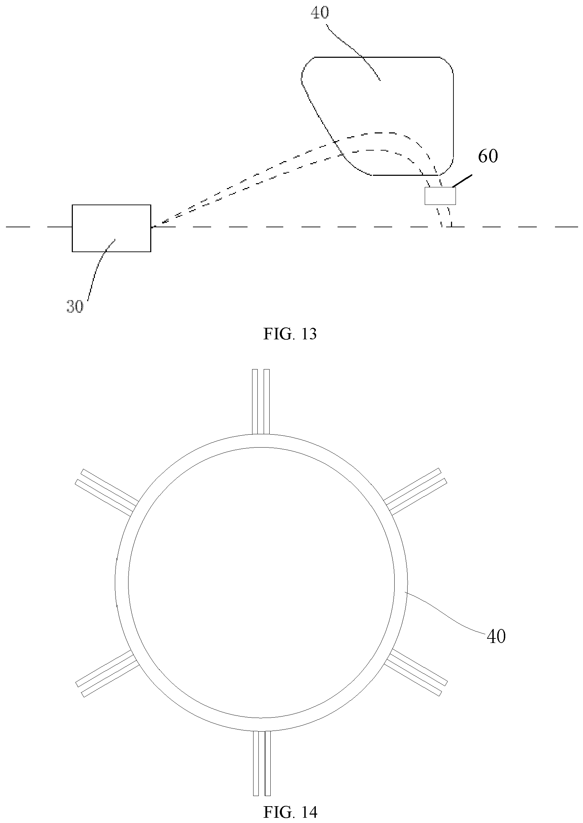

In addition, to implement that a target region (for example, an isocenter position) is adjustable at which the high-energy electron beam is converged when the electron beam treatment device 900 is in operation, in addition to the foregoing structure of the first deflection member 30 with an adjustable electromagnetic field, a second deflection member 60 (referring to FIG. 13) may be disposed on a path emitted from the annular deflection mechanism 40. The second deflection member may also generate an electromagnetic field, and a generated magnetic field strength may be adjusted when the second deflection member is powered on, so as to adjust the target region. It should be noted that, when adjustment of the generated magnetic field strength when the second deflection member is powered on is used to implement adjustment of the target region, the generated magnetic field strength when the first deflection member 30 is powered on may not be adjustable. The second deflection member may also be specifically configured as a vector magnet or a deflection resonant cavity.

In an embodiment, the target region is adjustable, i.e., an irradiation direction of the electron beam towards the target region is changeable.

In an embodiment, an irradiation direction of the electron beam towards the target region may be substantially fixed.

Referring to FIG. 14, in some embodiments, the annular deflection mechanism 40 may be configured in a ring structure. In this way, the high-energy electron beam passing through the annular deflection mechanism 40 may be converged from different directions within 360° to the target region (for example, a same isocenter point, or different discrete positions), thereby further improving a therapeutic effect of the electron beam treatment device 900 on a tumor when in operation. It should be noted that the annular deflection mechanism 40 is not limited to the foregoing ring structure. One skilled in the art may set a ring angle of the annular deflection mechanism 40 to any other angle such as 300°, 270°, or 180°. The magnetic field in the annular deflection mechanism 40 may also be adjustable to achieve beam adjustment (e.g., direction adjustment, e.g., implementation of axial scanning therapy). The annular deflection mechanism 40 may be a regular shape, or an irregular shape. As examples, the annular deflection mechanism 40 may be in a ring shape, a pentagon shape, a hexagon shape, an octagon shape or other shape. The annular deflection mechanism 40 may be entirely annular or partially annular (e.g., merely a partial loop or a broken line). A partially annular deflection mechanism 40 may be rotated, thereby implementing a desired treatment (e.g., a 360-degree treatment).

The annular deflection mechanism 40 may include a normal temperature magnet and/or a superconducting magnet, so that when the annular deflection mechanism 40 is in operation, second deflection processing may be specifically performed on the high-energy electron beam by the magnetic field strength, so that the deflected high-energy electron beam can be converged to the target region. It should be noted that when the annular deflection mechanism 40 includes the normal temperature magnet, a magnetic field value of the normal temperature magnet may be less than 2T. When the annular deflection mechanism 40 includes the superconducting magnet, a magnetic field value of the superconducting magnet may be higher than that of the normal temperature magnet, and to meet a requirement of the annular deflection mechanism 40, a lowtemperature cooling system generally needs to be provided in the annular deflection mechanism 40, which may include an Ammonia refrigeration system, a halogenated hydrocarbon refrigeration system, or a refrigeration system with special refrigerant.

In an embodiment, the electron beam treatment device 900 may further include a movable supporting device 120 configured for supporting a target subject. The movable supporting device 120 may be configured to adjust a position of the target region relative to the annular deflection mechanism 40 during an electron beam flash therapy, so that the target region is irradiated by the high-energy electron beam from different directions.

In an embodiment, the electron beam treatment device 900 may further include a bed 50. The bed 50 may be disposed through the annular deflection mechanism 40. The target region formed after the annular deflection mechanism 40 converges the high-energy electron beam may be disposed in a region where the bed 50 is located. In this way, when the electron beam treatment device 900 is in operation, the patient may lie flat on the bed 50 for high-energy electron beam irradiation therapy.

In an embodiment, a position of the target region relative to the electron beam treatment device is capable of being changed during a treatment such as a flash therapy or a volumetric-modulated arc therapy (VMAT). The position of the target region relative to the electron beam treatment device is changed by adjusting a travel direction of the high-energy electron beam and/or adjusting a position of a supporting device on which the target region is located (e.g., the target region may be changed by moving the bed 50.).

In an embodiment, the movable supporting device 120 may be rotatable so that the target region is able to be irradiated by the high-energy electron beam from any angle.

In an embodiment, the electron beam generator 10 may be located at a fixed location for emitting the electron beam along a fixed direction.

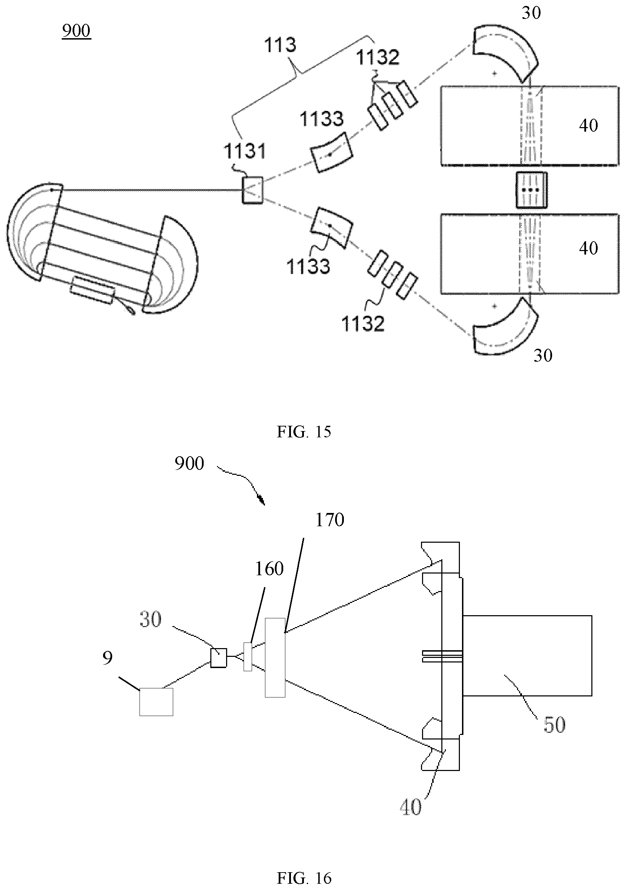

In an embodiment, referring to FIG. 15, the electron beam treatment device 900 may further include a beam regulator 113 configured to split the electron beam into a plurality of sub-beams transmitting along different directions. The electron beam treatment device 900 may include a plurality of groups of the first deflection member 30 and the annular deflection mechanism 40, which are configured to adjust the sub-beams, respectively, to generate a plurality of high-energy electron beams, and the plurality of high-energy electron beams are capable of irradiating the target region from different directions.

In an embodiment, the beam regulator 113 may further include a beam splitting element 1131 and a plurality of constraint elements 1132, the beam splitting element 1131 may be configured to split the electron beam into the plurality of sub-beams, and the plurality of constraint elements 1132 may be configured to constrain transmission directions of the plurality of sub-beams, so that each of the plurality of sub-beams enters into one of the plurality of groups of the first deflection member 30 and the annular deflection mechanism 40.

In an embodiment, referring to FIG. 16, the electron beam treatment device 900 may further include a converter 160 and a collimator 170, the converter 160 may be configured to convert the electron beam into a third radiation beam, and the collimator 170 may be configured to adjust the third radiation beam to align to the target region.

The present invention may further provide a method for electron beam flash therapy, including: emitting a high-energy electron beam by an electron beam emitter 9, directing the high-energy electron beam to a first deflection member 30, performing first deflection processing on the high-energy electron beam by the first deflection member 30, directing the high-energy electron beam processed by the first deflection processing to an annular deflection mechanism 40, and performing second deflection processing on the high-energy electron beam by the annular deflection mechanism 40, so that the high-energy electron beam is emitted towards a target region.

The present invention may further provide a system for flash therapy. The system includes an electron beam treatment device 900 configured to generate and deliver a high-energy electron beam that is applicable to flash therapy. The electron beam treatment device 900 includes an electron beam emitter 9, a first deflection member 30, and an annular deflection mechanism 40. The electron beam emitter 9 is configured to emit a high-energy electron beam. The first deflection member 30 is capable of performing first deflection processing on the high-energy electron beam. The annular deflection mechanism 40 is configured to receive the high-energy electron beam deflected by the first deflection member 30, and perform second deflection processing on the high-energy electron beam, so that the high-energy electron beam is emitted towards a target region.

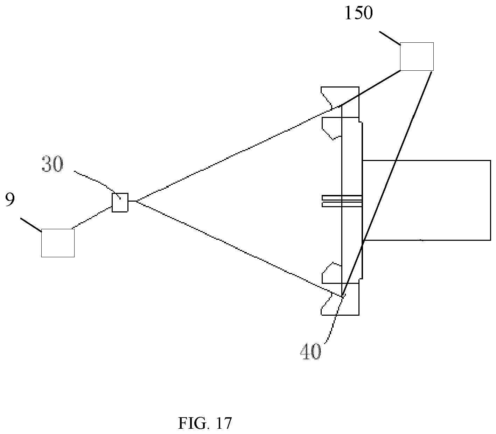

In an embodiment, referring to FIG. 17, the system may further include a second radiation treatment device 150 configured to emit a second radiation beam towards the target region for radiation treatment. The high-energy electron beam and the second radiation beam are configured for irradiating the target region from different directions.

In an embodiment, energy levels of the high-energy electron beam and the second radiation beam may be determined based on a position of the target region relative to the electron beam treatment device 900 and the second radiation treatment device 150.

In addition, the present invention may further provide a computer readable storage medium, and an instruction may be stored on the computer readable storage medium. When the instruction is executed by a processor, the following steps may be implemented: emitting a high-energy electron beam by an electron beam emitter 9, directing the high-energy electron beam to a first deflection member 30, performing first deflection processing on the high-energy electron beam by the first deflection member 30, directing the high-energy electron beam processed by the first deflection processing to an annular deflection mechanism 40, and performing second deflection processing on the high-energy electron beam by the annular deflection mechanism 40, so that the high-energy electron beam is emitted towards a target region.

One skilled in the art may understand that all or a part of the processes in the methods in the foregoing embodiments may be implemented by a computer program instructing related hardware. The computer program may be stored in a non-volatile computer readable storage medium. When the computer program is executed, the processes in the foregoing methods embodiments may be included. Any reference to a memory, a database, or another medium used in the embodiments provided in the present invention may include at least one of a non-volatile memory or a volatile memory. The non-volatile memory may include a Read-Only Memory (ROM), a magnetic tape, a floppy disk, a flash memory, an optical memory, a high-density embedded non-volatile memory, a Resistive Random Access Memory (ReRAM), a Magneto resistive Random Access Memory (MRAM), a Ferroelectric Random Access Memory (FRAM), a Phase Change Memory (PCM), a graphene memory, and the like. The volatile memory may include a Random Access Memory (RAM), an external cache, or the like. As an illustration and not a limitation, the RAM may be in multiple forms, such as a Static Random Access Memory (SRAM) or a Dynamic Random Access Memory (DRAM). The databases involved in the embodiments provided in the present invention may include at least one of a relational database or a non-relational database. The non-relational database may include a distributed database based on block chain or the like, which is not limited thereto. The processor in the embodiments provided in the present invention may be a general processor, a central processing unit, a graphics processor, a digital signal processor, a programmable logic device, a data processing logic device based on quantum computing, or the like, which is not limited thereto.

In conclusion, in the electron beam treatment device 900 of the present invention, various parts such as the electron cyclotron 20, the first deflection member 30, and the annular deflection mechanism 40 may cooperate with each other by an overall system design and an electron path design of the electron beam flashover device 100, so that a volume of the electron beam flashover device 100 is greatly reduced and manufacturing costs are reduced while flash therapy is performed by the high-energy electron beam, and flexibility of adjustment and control of the target position may be further implemented. In this way, it is convenient for a doctor to perform flash therapy to a patient.

Having thus described the basic concepts, it may be rather apparent to those skilled in the art after reading this detailed invention that the foregoing detailed invention is intended to be presented by way of example only and is not limiting. Various alterations, improvements, and modifications may occur and are intended to those skilled in the art, though not expressly stated herein. These alterations, improvements, and modifications are intended to be suggested by this invention, and are within the spirit and scope of the exemplary embodiments of this invention.

Moreover, certain terminology has been used to describe embodiments of the present invention. For example, the terms "one embodiment," "an embodiment," and/or "some embodiments" may mean that a particular feature, structure or characteristic described in connection with the embodiment is included in at least one embodiment of the present invention. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present invention.

Further, it will be appreciated by one skilled in the art, aspects of the present invention may be illustrated and described herein in any of a number of patentable classes or context including any new and useful process, machine, manufacture, or composition of matter, or any new and useful improvement thereof. Accordingly, aspects of the present invention may be implemented entirely hardware, entirely software (including firmware, resident software, micro-code, etc.) or combining software and hardware implementation that may all generally be referred to herein as a "unit," "module," or "system." Furthermore, aspects of the present invention may take the form of a computer program product embodied in one or more computer readable media having computer readable program code embodied thereon.

Furthermore, the recited order of processing elements or sequences, or the use of numbers, letters, or other designations therefore, is not intended to limit the claimed processes and methods to any order except as may be specified in the claims. Although the above invention discusses through various examples what is currently considered to be a variety of useful embodiments of the invention, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present invention, various features are sometimes grouped together in a single embodiment, figure, or description thereof for the purpose of streamlining the invention aiding in the understanding of one or more of the various inventive embodiments. This method of invention, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±1%, ±5%, ±10%, or ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. An electron beam treatment device (900), configured to generate and deliver a high-energy electron beam that is applicable to flash therapy, **characterized in that** the electron beam treatment device (900) comprises an electron beam emitter (9), a first deflection member (30), and an annular deflection mechanism (40); and wherein
the electron beam emitter (9) is configured to emit a high-energy electron beam;
the first deflection member (30) is configured to be capable of performing first deflection processing on the high-energy electron beam; and
the annular deflection mechanism (40) is configured to receive the high-energy electron beam deflected by the first deflection member (30), and perform second deflection processing on the high-energy electron beam, so that the high-energy electron beam is emitted towards a target region.

2. The electron beam treatment device (900) of claim 1, wherein energy of the high-energy electron beam is in a range of 100MeV to 200MeV.

3. The electron beam treatment device (900) of claim 1 or claim 2, wherein the electron beam emitter (9) comprises an electron beam generator (10) and an electron cyclotron (20);
the electron beam generator (10) is configured to generate an electron beam; and
the electron cyclotron (20) is configured to receive the electron beam from the electron beam generator (10), and perform cyclotron acceleration processing on the electron beam, so that the electron beam is formed into the high-energy electron beam.

4. The electron beam treatment device (900) of claim 3, wherein the electron cyclotron (20) comprises an acceleration cavity (21) and two groups of vector magnets (22), the two groups of vector magnets (22) are symmetrically disposed at both sides of the acceleration cavity (21) and are configured to guide the electron beam emitted from the acceleration cavity (21) to re-enter the acceleration cavity (21), and the acceleration cavity (21) is capable of performing acceleration processing on the electron beam in the acceleration cavity (21).

5. The electron beam treatment device (900) of claim 3 or claim 4, wherein the electron cyclotron (20) comprises a lead-out magnet (23), the lead-out magnet (23) is disposed on a cyclic path of the electron beam in the electron cyclotron (20), the lead-out magnet (23) is configured to lead out the high-energy electron beam formed after acceleration, and a position of the lead-out magnet (23) is capable of being adjusted relative to the electron cyclotron (20).

6. The electron beam treatment device (900) of any one of claims 3 to 5, wherein the electron cyclotron (20) comprises at least one of a race-track microtron, a betatron, or a petal-shaped accelerator.

7. The electron beam treatment device (900) of any one of claims 1 to 6, wherein a generated magnetic field strength of the first deflection member (30) is capable of being adjusted when the first deflection member (30) is powered on, so that the high-energy electron beam passing through the first deflection member (30) is capable of entering the annular deflection mechanism (40) at different angles.

8. The electron beam treatment device (900) of any one of claims 1 to 7, further comprising a second deflection member, wherein the second deflection member is configured to receive the high-energy electron beam emitted from the annular deflection mechanism (40) and perform third deflection processing on the high-energy electron beam, and a generated magnetic field strength of the second deflection member is capable of being adjusted when the second deflection member is powered on.

9. The electron beam treatment device (900) of any one of claims 1 to 8, wherein the first deflection member (30) comprises either or both of a vector magnet and a deflection resonant cavity.

10. The electron beam treatment device (900) of any one of claims 1 to 9, further comprising a movable supporting device (120) configured for supporting a target subject, wherein the movable supporting device (120) is configured to adjust a position of the target region relative to the annular deflection mechanism (40) during an electron beam flash therapy.

11. The electron beam treatment device (900) of any one of claims 1 to 10, further comprising a beam regulator (113) configured to split the electron beam into a plurality of sub-beams transmitting along different directions,
wherein the electron beam treatment device (900) comprises a plurality of groups of the first deflection member (30) and the annular deflection mechanism (40), which are configured to adjust the sub-beams, respectively, to generate a plurality of high-energy electron beams, and the plurality of high-energy electron beams are capable of irradiating the target region from different directions.

12. The electron beam treatment device (900) of claim 11, wherein the beam regulator (113) further comprises a beam splitting element (1131) and a plurality of constraint elements (1132),
the beam splitting element (1131) is configured to split the electron beam into the plurality of sub-beams; and
the plurality of constraint elements (1132) are configured to constrain transmission directions of the plurality of sub-beams, so that each of the plurality of sub-beams enters into one of the plurality of groups of the first deflection member (30) and the annular deflection mechanism (40).

13. The electron beam treatment device (900) of any one of claims 1 to 12, further comprising a converter (160) and a collimator (170), wherein the converter (160) is configured to convert the electron beam into a third radiation beam, and the collimator (170) is configured to adjust the third radiation beam to align to the target region.

14. A method for electron beam flash therapy, **characterized by** comprising:
emitting a high-energy electron beam by an electron beam emitter (9);
directing the high-energy electron beam to a first deflection member (30), and performing first deflection processing on the high-energy electron beam by the first deflection member (30); and
directing the high-energy electron beam processed by the first deflection processing to an annular deflection mechanism (40), and performing second deflection processing on the high-energy electron beam by the annular deflection mechanism (40), so that the high-energy electron beam is emitted towards a target region.

15. A system for flash therapy, **characterized by** comprising:
an electron beam treatment device (900) configured to generate and deliver a high-energy electron beam that is applicable to flash therapy,
wherein the electron beam treatment device (900) comprises an electron beam emitter (9), a first deflection member (30), and an annular deflection mechanism (40); and
wherein the electron beam emitter (9) is configured to emit a high-energy electron beam;
the first deflection member (30) is configured to be capable of performing first deflection processing on the high-energy electron beam; and
the annular deflection mechanism (40) is configured to receive the high-energy electron beam deflected by the first deflection member (30), and perform second deflection processing on the high-energy electron beam, so that the high-energy electron beam is emitted towards a target region.
